# EUROPEAN PATENT APPLICATION

(11) **EP 3 257 866 A1**
(43) Date of publication of application: **20.12.2017**
(21) Application number: 16175154.0
(22) Date of filing: 17.06.2016
(51) Int. Cl.: C07K 16/24

(54) **MODIFIED ANTI-TNF ANTIBODY AND USE THEREOF IN THE TREATMENT OF IBD**

(71) Applicant: Academisch Medisch Centrum, 1105 AZ Amsterdam (NL); Stichting Sanquin Bloedvoorziening, 1066 CX Amsterdam (NL)
(72) Inventor: The designation of the inventor has not yet been filed
(74) Representative: de Pauw, Elmar Sebastian David

(57) **Abstract**

The invention relates to modified anti-TNF antibody having an affinity for the 158V polymorfism of FcγRIIIa of more than 7.7 * 10⁶ KA (M⁻¹) as determined using biosensor analysis.

## Description

### ABSTRACT

The present invention provides modified anti-TNF antibodies having an enhanced affinity for the FcγRIIIa or non-fucosylated therapeutic antibodies. More particularly, the invention provides non-fucosylated anti-TNF antibodies, which show enhanced effectiveness in the treatment of IBD. Also provided are cells expressing the non-fucosylated anti-TNF antibodies and therapeutic methods using the non-fucosylated anti-TNF antibodies.

### BACKGROUND

The first successful treatment of a patient with Crohn's disease with anti-TNF in 1993 was the beginning of a breakthrough in the treatment of inflammatory bowel disease.(1) This has led to the development of several TNF neutralising drugs. So far, treatment with the IgG1 anti-TNF antibodies infliximab and adalimumab has shown to induce complete mucosal healing in a considerable proportion of patients with Crohn's disease (2, 3). However, despite their therapeutic efficacy, more than one-third of patients show no response to induction therapy (primary nonresponders), and in up to 50% of responders, TNF antagonist therapy becomes ineffective over time (secondary nonresponders). Thus, a critical need exists to develop new approaches and strategies that further optimize the efficacy of these drugs.
It is an object of the invention to improve the therapeutic efficacy of anti-TNF antibodies in the treatment of IBD.

### SUMMARY OF THE INVENTION

The present disclosure relates to variants of the anti-TNF antibody which are more effective in the treatment of IBD. The invention is based on the surprising finding that afucosylated anti-TNF antibodies display an enhanced induction of regulatory macrophages resulting in an improved therapeutic efficacy in the treatment of IBD compared to fucosylated anti-TNF antibodies. Antibodies of the IgG isotype bear two N-linked biantennary complex-type oligosaccharides attached to the antibody constant region (Fc) at position 297, in which the majority of the oligosaccharides (the Fc-glycan moiety) are core fucosylated. The Fc portion exercises the effector functions of the IgG, including antibody-dependent cellular cytotoxicity (ADCC) and complement-dependent cytotoxicity through the interaction of the Fc with either leukocyte receptors (FcgammaRs, FcγR) or complement component 1 (C1q), respectively. The Fc-glycan has a complex bi-antennary structure and is composed of N-acetylglucosamine (GlcNAc)- and mannose groups. The core structure can be further extended with galactose, sialic acid, fucose and an additional bisecting GlcNAc (bisection). The relative levels of some of these glycoforms vary greatly between individuals, but also with age, infection and pregnancy. The level of glycosylation is dependent on expression-levels of glycosyltransferases and glycosidases in the secretion pathway of the antibody producing plasma B cell. The fucosylation and bisection of IgG is generally very stable, with ∼95% serum IgG being fucosylated and ∼15% bisected. However, galactose is much more variable, being absent or present on one or both arms of the bi-antennary glycan (G0, G1 and G2, respectively). Similarly, sialic acid can be terminally added to the galactose, if present, referred to as S1 and S2. In normal healthy young individuals, ∼40% of serum IgG-Fc is galactosylated and ∼4% sialylated.

It has previously been found that the absence of IgG-Fc core fucose increases binding to FcγRIII by several fold, a finding increasingly being used to improve therapeutic antibodies, whose mode of action depends on FcγRIII binding. It has further been shown that the presence of the bisecting GlcNAc enhances antibody-dependent cell-mediated cytotoxicity (ADCC), presumably through inhibiting the incorporation of fucose. Recently, therapeutic antibodies with decreased fucosylation have been shown to improve overall survival as well as time to disease progression in a variety of human malignancies, such as breast, colon and haematological cancers, and genetic analysis of FcyR polymorphisms of cancer patients has demonstrated that ADCC is a major antineoplasm mechanism responsible for clinical efficacy.

However, the cellular and molecular mechanisms of actions of the anti-TNF antibodies in the treatment of IBD remain unknown (Oikonomopoulos A. et al., Curr Drug Targets. 2013 Nov;14(12):1421-32), and there is no indication that the mode of action relies on ADCC or works via binding of the FcγRIII. On the contrary, it is believed that infliximab and adalimumab exert their therapeutic effect in IBD by inducing M2-type wound-healing macrophages, which may make an important contribution to complete mucosal healing. This might indicate that other mechanisms than the induction of cytoxicity play a role. It is therefore surprising that afucosylated anti-TNF antibodies have superior properties in a treatment of IBD.

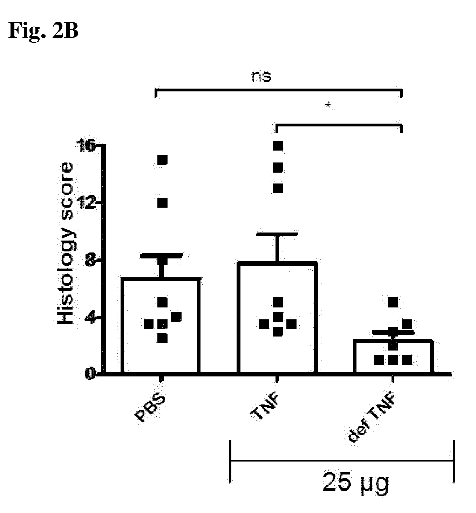

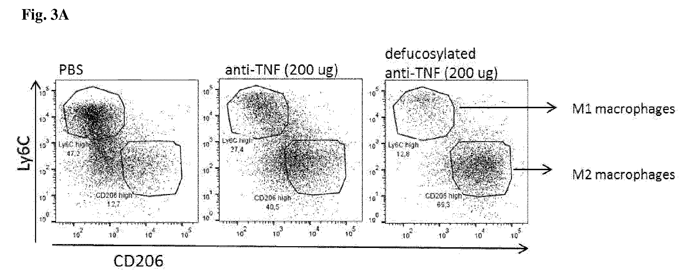

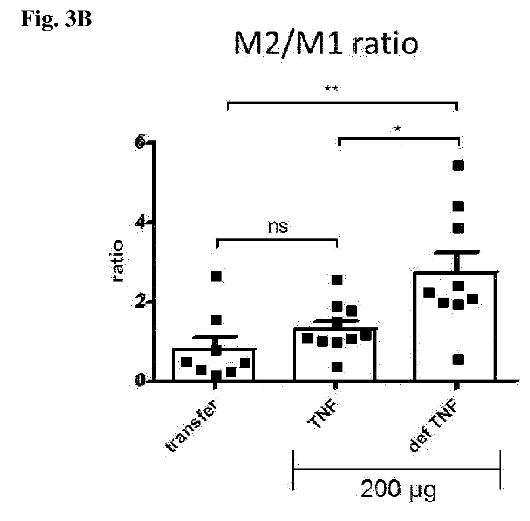

The inventors have demonstrated in mice, using a low dose, that afucosylated anti-TNF significantly reduced the histopathological disease severity compared to PBS treated animals (see Fig. 2 B). In in vitro experiments, it was shown that afucosylated adalimumab induced significantly more regulatory CD14+CD206+ macrophages than fucosylated adalimumab in a dose dependent manner. Furthermore, the inventors herein show that afucosylated mouse anti-TNF is more potent in inducing CD206+ regulatory macrophages in vivo (see Fig. 3).

Without wishing to be bound by theory, the inventors believe that the improved efficacy of these antibodies is caused by the enhanced affinity of the Fc domain of said antibodies for the FcγRIIIa receptor.

The invention therefore provides a modified anti-TNF antibody having an affinity for the 158V polymorfism of FcγRIIIa of more than 7.7 * 10⁶ KA (M⁻¹) as determined using biosensor analysis.

In a preferred embodiment, said modified anti-TNF antibody is an afucosylated anti-TNF antibody with an amount of fucose of 60% or less of the total amount of oligosaccharides (sugars) at Asn297. In a preferred embodiment, said Asn297 is high-galactosylated. This further improves the efficacy of the afucosylated anti-TNF antibody.

In a preferred embodiment, said afucosylated anti-TNF antibody induces a greater number of regulatory macrophages than a control fucosylated anti-TNF antibody. Preferably, said control fucosylated anti-TNF antibody is adalumimab.
In a preferred embodiment, said afucosylated anti-TNF antibody comprises complementarity determining regions (CDRs) of the antibody adalimumab.

Preferably, said anti-TNF antibody is selected from the group consisting of adalimumab, etanercept, golimumab, certolizumab and infliximab and a biosimilar form of any of adalimumab, etanercept, golimumab, and certolizumab and infliximab. T

he invention further provides a pharmaceutical composition for use according to the invention comprising the afucosylated anti-TNF antibody as defined above and a pharmaceutically acceptable carrier.

In a preferred embodiment, the amount of modified anti-TNF antibody according to the invention or the afucosylated anti-TNF antibody is at least 25%, 30%, 35%, 40%, 50%, 60%, 70%, 80%, 90%, 95%, 99%, 100% of the total number of anti-TNF antibodies.
The invention further provides a host cell expressing the modified anti-TNF antibody according to the invention or the afucosylated anti-TNF antibody as defined above.

The invention further provides said modified anti-TNF antibody or afucosylated anti-TNF antibody for use in the treatment of a disease.
Further, the invention provides modified anti-TNF antibody or afucosylated anti-TNF antibody for use in the treatment of inflammatory bowel disease (IBD).
In a preferred embodiment, said treatment comprises the administration of said modified anti-TNF antibody or afucosylated anti-TNF antibody between once or twice weekly and once every 10 weeks, preferably twice in the week.

The invention further provides a method for preparing an afucosylated anti-TNF antibody as defined above, the method comprising the steps of expressing the anti-TNF antibody in a host cell and harvesting, purifying and/or isolating said afucosylated anti-TNF antibody, wherein said host cell has a reduced capacity to fucosylate or wherein said host cell expresses said anti-TNF antibody in the presence of an effective amount of a fucosylation inhibitor. Said host cell expresses constitutively or transiently with a nucleic acid molecule encoding an anti-TNF antibody amino acid sequence.

In a preferred embodiment, said host cell expresses beta-1,4-mannosyl-glycoprotein 4-beta-N-acetylglucosaminyltransferase (GntIII) or GDP-6-deoxy-D-lyxo-hexulose reductase (RMD).

Preferably, said fucosylation inhibitor is a fucose analog. Preferably, said fucose analog is 2-deoxy-2-fluoro-1-fucose.

### BRIEF DESCRIPTION OF THE DRAWINGS

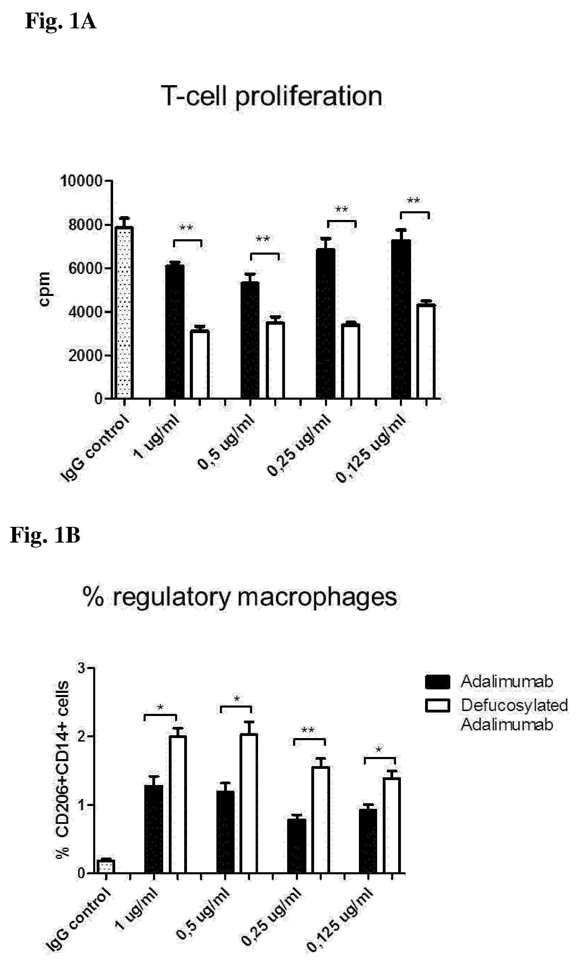

Fig. 1 shows that reduced fucosylation of adalimumab enhances immunosuppressive properties of the antibody in vitro. MLRs were treated with a serial dilution of adalimumab or afucosylated adalimumab. Human IgG was added to every condition to reach a total antibody concentration of 1 µg/ml per well. (A) Proliferation was measured by thymidine incorporation. Afucosylated adalimumab was significantly more potent in inhibiting T-cell proliferation in a dose dependent manner. (B) MLRs were stained for CD14-PE and CD206-APC and analyzed by flow cytometry for percentages of CD14+CD206+ cells. Afucosylated adalimumab induced significantly more CD14+CD206+ macrophages than fucosylated adalimumab in a dose dependent manner Bars represent mean ± standard error of the mean. Statistical significance was analyzed Mann Whitney test (* = p < 0.05, ** = p < 0.01).

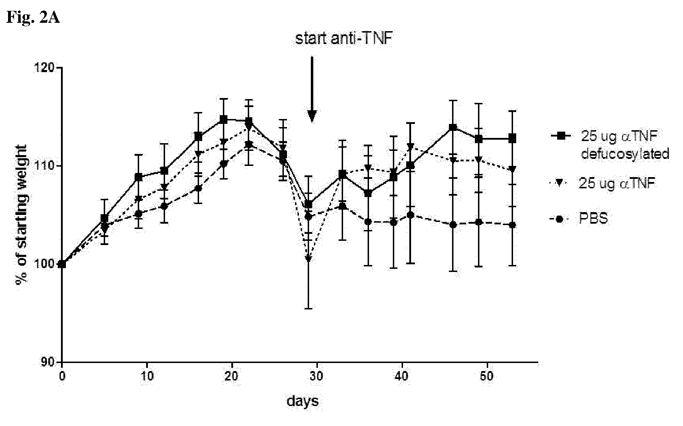

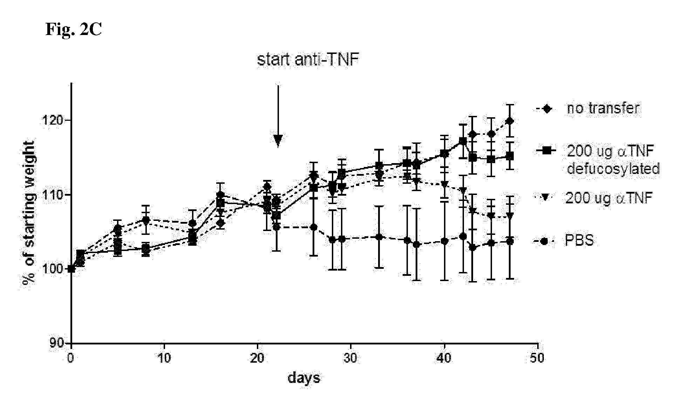

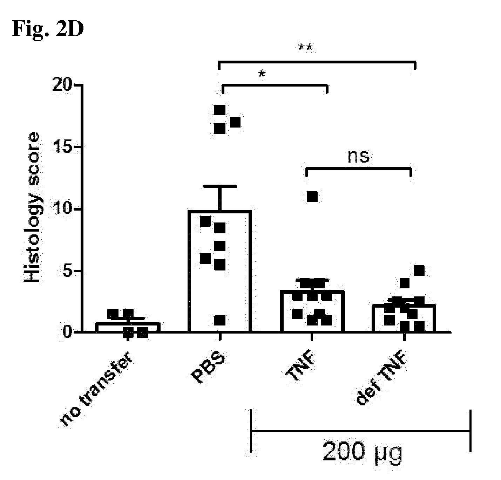

Fig. 2 shows that afucosylated mouse anti-TNF is more effective in the T-cell transfer model of colitis. (A) Body weight of Balb/c SCID mice after transfer with CD4+CD45RB^{hi} cells. Treatment groups contained 7-8 mice per group. Animals were treated with PBS or a low dose (25 µg) of mouse anti-TNF or afucosylated mouse anti-TNF twice weekly. (B) Histopathological severity of the colitis. With a suboptimal dose of 25 µg, only afucosylated anti-TNF significantly reduced the histopathological disease severity compared to PBS treated animals. (C) Body weight of mice in T-cell transfer experiment where animals were treated with PBS or a high dose (200 µg) of mouse anti-TNF or afucosylated mouse anti-TNF twice weekly. Groups consist of 9-10 mice per group. Naive group did not receive T-cell transfer and contains 5 mice. (D) Histopathological severity of the colitis. At the high dose of 200 µg, both anti-TNFs significantly improved colitis severity. Bars represent mean ± standard error of the mean. Statistical significance was determined with a Kruskal-Wallis test followed by Dunn's post-hoc analysis. (* = p < 0.05, *** = p < 0.001)
Fig 3 shows that afucosylated mouse anti-TNF is more potent in inducing CD206+ regulatory macrophages in vivo. (A) Colons from mice with transfer colitis that received the high dose of 200 µg anti-TNF were analyze by flow cytometry. Representative flow cytometry plots are shown of macrophage subsets after gating for CD45+CD11b+Ly6G-CD64+ myeloid cells. (B) CD206^{hi}Ly6C^{lo} macrophages and CD206^{lo}Ly6C^{hi} were gated as shown in figure 3A and ratios were calculated. Afucosylated anti-TNF induced significantly more CD206+ regulatory macrophages in the colons of mice with transfer colitis. Bars represent mean ± standard error of the mean. Statistical significance was determined with a Tukey's Multiple Comparison Test. (* = p < 0.05, *** = p < 0.001)

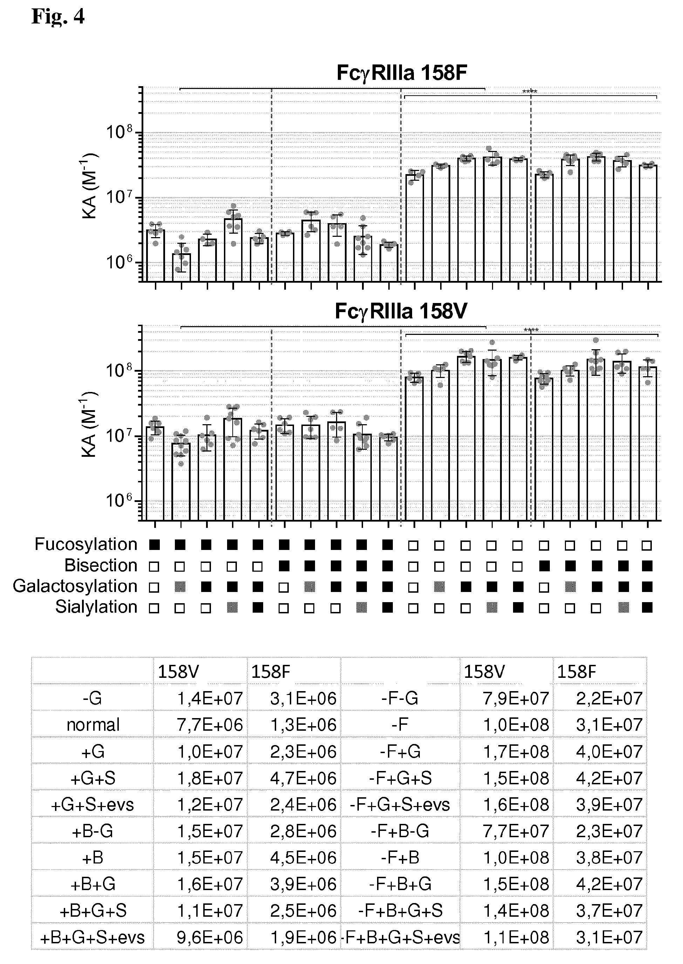

Fig. 4 shows binding affinities of glyco engineered human IgG1 produced in HEK cells with low (open symbols on x-axis), intermediary (gray symbol on x-axis), or high (closed symbols) levels for fucosylation, bisection, galactosylation or sialiltion in any possible combination to human FcγRIIIa of the two known allotypic forms (F/V158). Binding is clearly elevated for non-fucosylated, but in particularly non-fucosylated and highly galacotylated (p<0.0001, note logarithmic scale), with sialylation on top of galactosylation having slight negative effect.
   -G = low-galactosylation; normal = adalumimab; +G = high Galactosylation; +S = intermediate sialylation; +evs = high sialylation; -F = low fucosylation; +F high fucosylation; +B high Bidisection.

### DETAILED DESCRIPTION

The inventors have found that afucosylated anti-TNF antibodies have an improved efficacy in the treatment of IBD compared to fucosylated anti-TNF antibodies. Without wishing to be bound by theory, the inventors believe that the improved efficacy of these antibodies is caused by the enhanced affinity of the Fc domain of said antibodies for the FcγRIIIa receptor. The FcγRIIIa receptor is involved in ADCC response, and it has not been shown that it plays any therapeutically relevant role in the treatment of IBD. Therefore, it is surprising that anti-TNF antibodies having an enhanced affinity for a FcγRIIIa receptor are superior to existing anti-TNF antibodies which are used in the treatment of IBD.

The FcγRIIIa receptor is expressed on natural killer (NK) cells and macrophages. Genomic polymorphism of the FcγRIIIa receptor corresponding to phenotypic expression of valine (V) or phenylalanine (F) at position 158 influences the binding of IgG1 to this receptor.
The term "158V polymorphism of FcγRIIIa" as used herein refers to a variant of the FcγRIIIa encoded by the nucleic acid sequence CTGAAGACACATTTTTACTCCCAAC (SEQ ID NO: 1).

The invention provides modified anti-TNF antibodies having an enhanced affinity for the FcγRIIIa receptor. The term " affinity" as used herein refers to the strength of interaction between an the Fc receptor and the anti-TNF antibody. The affinity is expressed as an affinity constant KA.

The affinity constant is defined as KA = [AB] / [A] [B], wherein A denotes the FcγRIIIa receptor and B the anti TNF antibody, the concentration of complex AB is proportional to the concentrations of the reactants and to a constant property of the two reactants for given reaction conditions of temperature, ionic strength and pH.
In other words, KA describes how much antibody- FcyRIIIa receptor complex exists at the point when equilibrium is reached. The time taken for this to occur depends on rate of diffusion and is similar for every antibody. However, high-affinity antibodies for FcγRIIIa receptors will bind a greater amount of FcγRIIIa receptors in a shorter period of time than low-affinity antibodies.

Known anti-TNF antibodies such as adalimumab have an affinity constant for the 158V variant of FcγRIIIa of 7.7 * 10⁶ KA (M⁻¹) and 1.35 * 10⁶ KA (M⁻¹) for the 158F variant, as determined by biosensor analysis as described herein. Therefore, the invention encompasses any anti-TNF antibody having an affinity of more than 7.7 * 10⁶ KA (M⁻¹) for the 158V variant of FcγRIIIa or of more than 1.35 * 10⁶ KA (M⁻¹) for the 158F variant of FcγRIIIa. In a preferred embodiment, said anti-TNF antibody has an affinity of at least 1.0 * 10⁷ KA (M⁻¹), 2.0* 10⁷ KA (M⁻¹), 3.0 * 10⁷ KA (M⁻¹), 4.0 * 10⁷ KA (M⁻¹), 5.0 * 10⁷ KA (M⁻¹), 6.0 * 10⁷ KA (M⁻¹), 7.0 * 10⁷ KA (M⁻¹), 8.0 * 10⁷ KA (M⁻¹), 9.0 * 10⁷ KA (M⁻¹), 1.0 * 10⁸ KA (M⁻¹), 1.1 * 10⁸ KA (M⁻¹), 1.2 * 10⁸ KA (M⁻¹), 1.3 * 10⁸ KA (M⁻¹), 1.4 * 10⁸ KA (M⁻¹), 1.48 * 10⁸ KA (M⁻¹), or 1.5 * 10⁸ KA (M⁻¹), for the 158V variant of FcγRIIIa or of at least 2.0 * 10⁶ KA (M⁻¹), 3.0 * 10⁶ KA (M⁻¹), 4.0 * 10⁶ KA (M⁻¹), 5.0 * 10⁶ KA (M⁻¹), 6.0 * 10⁶ KA (M⁻¹), 7.0 * 10⁶ KA (M⁻¹), 8.0 * 10⁶ KA (M⁻¹), 9.0 * 10⁶ KA (M⁻¹), 1.0 * 10⁷ KA (M⁻¹), 3.06 * 10⁷ KA (M⁻¹), 3.11 * 10⁷ KA (M⁻¹), 4.00 * 10⁷ KA (M⁻¹), or 4.24 * 10⁷ KA (M⁻¹), for the 158F variant of FcγRIIIa.

As methods of determining the affinity of an antibody can have a great influence on the absolute value of the association constant of an antibody for a certain Fc receptor, it is required that said affinity is determined under comparable conditions and using the same or comparable methods. Therefore, the affinity constant must be determined using biosensor analysis, using the same pH, temperature and buffer composition as described herein.

It is known in the art how antibodies with a greater affinity for a FcγRIIIa receptor can be produced. The affinity of an antibody for known FcγRIIIa variants are known to be dependent on for instance polymorphism in the Fc portions of the antibody and glycosylation of the glycan moiety at Asn297 of the antibody. An overview of these polymorphisms and glycosylations which enhance the affinity of antibodies for the FcγRIIIa is provided for instance in Table 5 of Bruhns et al., Immun. Rev 2015, vol. 268, 25-51. The mutations enhancing the affinity for the FcγRIIIa V158 and F158 (indicated by "**↗**", "**↗↗**" or "**↗↗↗**") are incorporated by reference herein.

In a preferred embodiment, said anti-TNF antibody having an enhanced affinity for a FcγRIIIa receptor is wherein the glycan moiety at Asn297 of the antibody is afucosylated.
As used herein the term "glycan moiety" with respect to an antibody refers to the glycan moiety linked to the asparagine at amino acid position 297 of the antibody.

The term "afucosylated antibody" or "non-fucosylated antibody" refers to an antibody of IgG1, lgG2, lgG3, or lgG4 isotype with an altered pattern of glycosylation in the Fc region at Asn297 having a reduced level of fucose residues. Glycosylation of human IgG1, lgG2, lgG3, or lgG4 occurs at Asn297 as core fucosylated bianntennary complex oligosaccharide glycosylation terminated with up to 2 Gal residues. These structures are designated as G0, G1 (α1,6 or α1,3) or G2 glycan residues, depending from the amount of terminal Gal residues (Raju, T. S., BioProcess Int. 1 (2003) 44-53). CHO type glycosylation of antibody Fc parts is e.g. described by Routier, F. H., Glycoconjugate J. 14 (1997) 201-207. Antibodies which are recombinantly expressed in non glycomodified CHO host cells usually are fucosylated at Asn297 in an amount of at least 85%. It should be understood that the term an afucosylated antibody as used herein includes an antibody having no fucose in its glycosylation pattern. It is commonly known that typical glycosylated residue position in an antibody is the asparagine at position 297 according to the EU numbering system ("Asn297").

The "EU numbering system" or "EU index" is generally used when referring to a residue in an immunoglobulin heavy chain constant region (e.g., the EU index reported in Kabat et al., Sequences of Proteins of Immunological Interest, 5th Ed. Public Health Service, National Institutes of Health, Bethesda, Md. (1991) expressly incorporated herein by reference).

Thus an afucosylated antibody according to the invention means an antibody of IgG1, lgG2, lgG3, or lgG4 isotype wherein the amount of fucose is 60% or less of the total amount of oligosaccharides (sugars) at Asn297 (which means that at least 40% or more of the oligosaccharides of the Fc region at Asn297 are afucosylated). In one embodiment the amount of fucose is between 40% and 60% of the oligosaccharides of the Fc region at Asn297. In another embodiment the amount of fucose is 50% or less, and in still another embodiment the amount of fucose is 30% or less of the oligosaccharides of the Fc region at Asn297. In an alternative embodiment, the amount of fucose is 0% of the oligosaccharides of the Fc region at Asn297. According to the invention "amount of fucose" means the amount of said oligosaccharide (fucose) within the oligosaccharide (sugar) chain at Asn297, related to the sum of all oligosaccharides (sugars) attached to Asn 297 (e.g. complex, hybrid and high mannose structures) measured by MALDI-TOF mass spectrometry and calculated as average value (a detailed procedure to determine the amount of fucose, is described e.g. in WO 2008/077546). Furthermore, in one embodiment, the oligosaccharides of the Fc region are bisected.

As used here the term "anti-TNF antibody" refers broadly to any antibody having specificity for human TNF-a, including but not limited to adalimumab, etanercept, golimumab, and infliximab and a biosimilar form of adalimumab, etanercept, golimumab, and infliximab, and the anti-TNF antibodies as described in EP 2419448 A1. The term "antibody" encompasses the various forms of antibodies including but not being limited to whole antibodies, human antibodies, humanized antibodies and genetically engineered antibodies like monoclonal antibodies, chimeric antibodies or recombinant antibodies as well as fragments of such antibodies as long as the characteristic properties according to the invention are retained. It is essential that said anti-TNF antibody comprises a FcγRIIIa binding portion, preferably an Fc domain, more preferably a human Fc domain. More preferably, said antibody comprises an Fc domain of an isotype selected from the group consisting of human IgG1, IgG2, IgG3, and IgG4. More preferably, said antibody comprises an Fc domain of human IgG1. Said antibody is preferably a monoclonal antibody, preferably a recombinant monoclonal antibody. In one particular embodiment, said antibody comprises the heavy chain CDRs or complete VH domain amino acid sequence of infliximab, adalimumab, certolizumab, or golimumab. In one particular embodiment, VDL comprises the light chain CDRs or complete VL domain amino acid sequence of infliximab, adalimumab, certolizumab pegol, or golimumab. In a preferred embodiment, said anti-TNF antibody comprises complementarity determining regions (CDRs) of the antibody adalimumab.

As used herein, the term "biosimilar" is used in a manner that is consistent with the working definition promulgated by the US FDA which defines a biosimilar product to be one that is "highly similar" to a reference product (despite minor differences in clinically inactive components). In practice there can be no clinically meaningful differences between the reference product and the biosimilar product in terms of safety, purity, and potency (Public Health Service (PHS) Act §262). For example, a biosimilar form of adalimumab is an antibody which a regulatory authority deems to be "highly similar" to the reference product Humira® on the basis of an abbreviated regulatory submission. In a highly preferred embodiment, said anti-TNF antibody is adalimumab.

As used herein, the term " adalimumab " refers to the anti-TNF antibody marketed as HUMIRA™, having Chemical Abstracts Service (CAS) designation 331731-18-1.
As used herein, the term "infliximab" refers to the anti-TNF antibody marketed as REMICADE™, having Chemical Abstracts Service (CAS) designation 170277-31-3.
As used herein, the term " golimumab" refers to the anti-TNF antibody marketed as SIMPONI™, having Chemical Abstracts Service (CAS) designation 476181-74-5.

The term "human TNF-a " (abbreviated herein as hTNF- alpha, TNFcc, or simply hTNF), as used herein, is intended to refer to a human cytokine that exists as a 17 kDa secreted form and a 26 kDa membrane associated form, the biologically active form of which is composed of a trimer of noncovalently bound 17 kDa molecules. The structure of hTNF-alpha is described further in, for example, Pennica, D., et al. (1984) Nature 312:724-729; Davis, J. M., et al. (1987) Biochem 26: 1322-1326; and Jones, E. Y., et al. (1989) Nature 338:225-228.

In a preferred embodiment, said glycan moiety at Asn297 of the modified anti-TNF antibody or the afucosylated anti-TNF antibody of the invention is hyper-galactosylated (or " high-galactosylated" ). Without wishing to be bound by theory, the inventors believe that this further enhances the efficacy of said modified or afucosylated anti-TNF antibody in the treatment of IBD through enhanced binding to FcγRIIIa. Methods of producing hyper-galactosylated antibodies are known in the art, and disclosed for instance in US20140296490. In an embodiment, the method comprises providing a host cell with a nucleic acid molecule, such as a plasmid, encoding an antibody amino acid sequence, allowing the host cell to translate the nucleic acid molecule in the presence of 2-deoxy-d-galactose, thereby producing antibodies with enhanced galactosylation in the glycan moiety. 2-doxy-d-galactose acts as a decoy substrate for galactosylation, resulting in the production of antibodies comprising a glycan moiety that is high-galactosylated.
Thus an afucosylated antibody according to the invention means an antibody of IgG1, lgG2, lgG3, or lgG4 isotype wherein the amount of galactose is 25%, 30%, 35, 40%, 45%, 50% or higher of the total amount of oligosaccharides (sugars) at Asn297. In one embodiment the amount of galactose is higher than 60%. In another embodiment the amount of galactose is 70% or higher, and in still another embodiment the amount of galactose is 80% or higher of the oligosaccharides of the Fc region at Asn297. In an alternative embodiment, the amount of galactose is 100% of the oligosaccharides of the Fc region at Asn297.

In another preferred embodiment, said modified anti-TNF antibody or said afucosylated anti-TNF antibody is low-sialylated, which reduces the affinity for the FcγRIIIa receptor.

The term "low-sialylated" as used in accordance with the present invention refers to a reduced amount of sialic acid on the glycan moiety of Asn297. Preferably, said low-sialylated anti-TNF antibody comprises less than 30%, 20%, 10%, 5%, 4%, 3% or 2% of sialic acid at the glycan moiety of Asn297.

In another preferred embodiment, said modified anti-TNF antibody or said afucosylated anti-TNF antibody is hyper bisected (bisecting GlcNAc). The amount of bisecting GlcNAc is preferably higher than 25%. In another embodiment the amount of bisecting GlcNAc is 50% or higher, and in still another embodiment the amount of bisecting GlcNAc is 66% or higher of the oligosaccharides of the Fc region at Asn297.

In a preferred embodiment, said modified anti-TNF antibody or said afucosylated anti-TNF antibody is afucosylated, hyper-galactosylated and hyper-bidesected.

The invention further provides a modified anti-TNF antibody or afucosylated anti-TNF antibody for use in the treatment of a disease.
Further, the invention provides a modified anti-TNF antibody or afucosylated anti-TNF antibody for use in the treatment of inflammatory bowel disease (IBD).

As used herein, the phrase "inflammatory bowel disease (IBD)" refers to a disorder or disease characterized by inflammatory activity in the GI tract. IBD includes, but is not limited to, Crohn's disease, ulcerative colitis, Johne's disease, Behçet's syndrome, collagenous colitis, diversion colitis, indeterminate colitis, microscopic colitis, infective colitis, ischaemic colitis, lymphocytic colitis, idiopathic inflammation of the small and/or proximal intestine, IBD-related diarrhea and closely related diseases and disorders of the gastrointestinal tract.

The inventors have found that the afucosylated anti-TNF antibody of the invention induces regulatory macrophages, which are believed to play a crucial in the treatment of IBD. Therefore, in a preferred embodiment, said modified anti-TNF antibody or said afucosylated anti-TNF antibody induces a greater number of regulatory macrophages than a control fucosylated anti-TNF antibody.
The term "regulatory macrophage" as used herein refers to a macrophage that has an immunosuppressive function and is characterized by a specific expression pattern of certain marker genes, including CD14 and CD206.

The modified anti-TNF antibody or afucosylated anti-TNF antibodies according to the invention induce a higher number of regulatory macrophages compared anti-TNF antibodies having no reduced fucose, when using the same dose. Preferably, said induction is determined using the T-cell transfer model of colitis as described herein.
The number of regulatory macrophages may be determined using any method available. A suitable method to determine the number of regulatory macrophages is by determining the percentage of CD206+ regulatory macrophages within the MLR by flow cytometry.

### Pharmaceutical Compositions and Pharmaceutical Administration

The afucosylated anti-TNF antibodies of the invention can be incorporated into pharmaceutical compositions suitable for administration to a subject. Typically, the pharmaceutical composition comprises an afucosylated anti-TNF antibody of the invention and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable substances such as wetting or minor amounts of auxiliary substances such as wetting or emulsifying agents, preservatives or buffers, which enhance the shelf life or effectiveness of the afucosylated anti-TNF antibody.
In a preferred embodiment, the pharmaceutical composition for use according to the invention comprises an amount of the modified anti-TNF antibodies or afucosylated anti-TNF antibodies which is at least 25% of the total number of anti-TNF antibodies. Preferably, said amount is at least 30%, 35%, 40%, 50%, 60%, 70%, 75, 80%, 85%, 90%, 95% , 99% or 100%.

The compositions of this invention may be in a variety of forms. These include, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies. The preferred mode of administration is parenteral (e.g., intravenous, subcutaneous, intraperitoneal, intramuscular). In a preferred embodiment, the antibody is administered by intravenous infusion or injection. In another preferred embodiment, the antibody is administered by intramuscular or subcutaneous injection.

Pharmaceutical compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (i.e., afucosylated anti-TNF antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The modified anti-TNF or afucosylated antibodies of the present invention can be administered by a variety of methods known in the art, although for many therapeutic applications, the preferred route/mode of administration is intravenous injection or infusion. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J. R. Robinson, ed., Marcel Dekker, Inc., New York, 1978.

In certain embodiments, the modified anti-TNF antibody or afucosylated anti-TNF antibody of the invention may be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation.

Supplementary active compounds can also be incorporated into the compositions of the invention. In certain embodiments, a modified anti-TNF antibody or an afucosylated anti-TNF antibody of the invention is coformulated with and/or co-administered with one or more additional therapeutic agents. For example, an anti-hTNFα modiefied anti-TNF antibody or afucosylated anti-TNF antibody of the invention may be co-formulated and/or co-administered with one or more additional antibodies that bind other targets (e.g., antibodies that bind other cytokines or that bind cell surface molecules), one or more cytokines, soluble TNFα receptor (see e.g., PCT Publication No. WO 94/06476) and/or one or more chemical agents that inhibit hTNFα production or activity (such as cyclohexane-ylidene derivatives as described in PCT Publication No. WO 93/19751) or promote regulatory macrophage formation. Furthermore, one or more antibodies of the invention may be used in combination with two or more of the foregoing therapeutic agents. Such combination therapies may advantageously utilize lower dosages of the administered therapeutic agents, thus avoiding possible toxicities or complications associated with the various monotherapies.

The pharmaceutical compositions of the invention may include a "therapeutically effective amount" or a "prophylactically effective amount" of a modified anti-TNF antibody or an afucosylated anti-TNF antibody of the invention. A "therapeutically effective amount" refers to an amount effective, at dosages and for periods of time necessary, to achieve the desired therapeutic result. A therapeutically effective amount of the modified anti-TNF antibody or afucosylated anti-TNF antibody may vary according to factors such as the disease state, age, sex, and weight of the individual, and the ability of the modified anti-TNF antibody or afucosylated anti-TNF antibody to elicit a desired response in the individual. A therapeutically effective amount is also one in which any toxic or detrimental effects of the modified anti-TNF antibody or afucosylated anti-TNF antibody are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

An exemplary, non-limiting range for a therapeutically or prophylactically effective amount of an a modified anti-TNF antibody or afucosylated anti-TNF antibody of the invention is 0.1-20 mg/kg, more preferably 1,25 -10 mg/kg. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

### Methods for producing afucosylated antibodies and host cells for producing the fucosylated antibodies

The invention further provides a method for preparing an afucosylated anti-TNF antibody as defined above, the method comprising the steps
- providing a host cell with a nucleic acid molecule encoding an anti-TNF antibody amino acid sequence;
- allowing expression of the anti-TNF antibody in the host cell under conditions
wherein said afucosylated antibody is produced; and
- harvesting, purifying and/or isolating said afucosylated antibody,
Said conditions wherein said afucosylated antibody is produced are well known in the art. Suitable conditions are wherein said host cell has a reduced capacity to fucosylate, for example due to the lack of enzymatic machinery to fucosylate. Other known methods include methods wherein said host cell expresses said anti-TNF antibody in the presence of an effective amount of a fucosylation inhibitor.

Said fucosylation inhibitor may be any compound which inhibits the fucosylation of the glycan moiety at Asn297.
Said host cell having a reduced capacity to fucosylate is well known in the art. In order to decrease fucosylation, said host cells are host cells wherein one or more genes encoding enzymes catalyzing fucosylation are knocked-out. This is for instance achieved using artificial endonucleases that can be designed to bind to a specific genomic target site and induce a DNA break, such as with the CRISPR/Cas9 technology. Methods to knockout genes are well known in the art. In an embodiment, said host cell is a cell wherein α1,6-fucosyltransferase activity is decreased or eliminated according to U.S. Pat. No. 6,946,292 to generate modified host cell having a reduced capacity to add a fucose moiety to Asn297 of the anti-TNF antibody.
Alternatively, a host cell can be used that expresses beta-1,4-mannosyl-glycoprotein 4-beta-N-acetylglucosaminyltransferase (GntIII) or GDP-6-deoxy-D-lyxo-hexulose reductase (RMD). Suitably, the host cell is transfected with a nucleic acid encoding RMD. RMD blocks the de novo production of fucose in the host cell thereby limiting the availability of fucose for addition to the glycan moiety. In a preferred embodiment, said host cell expresses beta-1,4-mannosyl-glycoprotein 4-beta-N-acetylglucosaminyltransferase (GntIII). In another preferred embodiment, said host cell expresses GDP-6-deoxy-D-lyxo-hexulose reductase (RMD). Alternatively, a decreased amount of fucose at the glycan moiety at Asn297 in said host cell may also be achieved by expressing said anti-TNF antibody in the presence of an effective amount of a fucosylation inhibitor. Said fucosylation inhibitor may function as a decoy substrate, thereby limiting the amount of fucose added.
Preferably, said fucosylation inhibitor is a fucose analog. Preferably, said fucose analog is 2-deoxy-2-fluoro-1-fucose. Said host cell expresses constitutively or transiently with a nucleic acid molecule encoding an anti-TNF antibody amino acid sequence. The term "expresses constitutively or transiently" refers to the expression of said nucleic acid as a result of respectively, the expression of a nucleic acid integrated into the host cells genome or as the result of the expression of a nucleic acid which was introduced via transient transfection.
In a preferred embodiment, said host cell expresses GDP-6-deoxy-D-lyxo-hexulose reductase (RMD) and/or β-1,4-galactosyltransferase 1 (B4GALT1) and/or β-1,4-galactosyltransferase 2 (B4GALT2). In another preferred embodiment, said host cells is further cultured in the presence of 2-doxy-2-fluoro-d-galactose. In a preferred embodiment, said host cell further overexpresses β-1,4-galactosyltransferase. In a preferred embodiment, said host cell expresses GDP-6-deoxy-D-lyxo-hexulose reductase (RMD),and/or β-1,4-galactosyltransferase (e.g. B4GALT1). In another preferred embodiment, said host cells is further cultured in the presence of 2-deoxy-d-galactose.

Other glycoengineering methods yielding afucosylated antibodies according to the invention are described e.g. in Niwa, R., et al., J. Immunol. Methods 306 (2005) 151-160; Shinkawa, T., et al., J Biol Chem, 278 (2003) 3466-3473; WO 03/055993 or US2005/0249722.
The amount of antibody fucosylation can be predetermined e.g. either by culture conditions (e.g. culture time) or by combination of at least two antibodies with different fucosylation amount. Such afucosylated antibodies and respective glycoengineering methods are described in WO 2005/044859, WO 2004/065540, WO 2007/031875, Umana, P., et al., Nature Biotechnol. 17 (1999) 176-180, WO 99/154342, WO 2005/018572, WO 2006/116260, WO 2006/114700, WO 2005/011735, WO 2005/027966, WO 97/028267, US2006/0134709, US2005/0054048, US2005/0152894, WO 2003/035835, WO 2000/061739.

Said host cell is preferably a mammalian cell, preferably selected from Chinese Hamster Ovary (CHO cells) (including DHFR" CHO cells, described in Urlaub and Chasin, 1980, Proc. Natl. Acad. Sci. USA 77:4216-4220, used with a DHFR selectable marker, e.g., as described in Kaufman and Sharp, 1982, MoI. Biol. 159:601-621), NSO myeloma cells, COS cells, 293 cells and SP2/0 cells. In a preferred embodiment, HEK-293F FreeStyle cell are used.
When recombinant expression vectors encoding antibody genes are introduced into mammalian host cells, the antibodies are produced by culturing the host cells for a period of time sufficient to allow for expression of the antibody in the host cells or secretion of the antibody into the culture medium in which the host cells are grown. Antibodies can be recovered from the culture medium using standard protein purification methods. Host cells can also be used to produce portions of intact antibodies. It is understood that variations on the above procedure are within the scope of the present disclosure. For example, it can be desirable to transfect a host cell with DNA encoding either the light chain or the heavy chain of an afucosylated anti-TNF antibody of this disclosure.

Features may be described herein as part of the same or separate aspects or embodiments of the present invention for the purpose of clarity and a concise description. It will be appreciated by the skilled person that the scope of the invention may include embodiments having combinations of all or some of the features described herein as part of the same or separate embodiments.
The invention will be explained in more detail in the following, non-limiting examples.

### EXAMPLE

### Methods

### Generation of afucosylated anti-TNF

We generated fucosylated and afucosylated IgG1 anti-human TNF (adalimumab) and chimeric human IgG1 anti-mouse TNF (8c11), for in vitro and in vivo use, respectively. DNA for the heavy and light chain variable regions of adalimumab (patent US 6090382) and 8C11 monoclonal antibody anti-mouse TNF were designed with compatible overhangs for our human IgG1-expressing constructs (15, 16) and then synthesized.
The resulting constructs were then expressed in HEK-293F FreeStyle cell line expression system (Life Technologies) with co-transfection of vectors encoding p21, p27 and pSVLT genes to increase protein production.(17) To generate antibodies with lower core-fucosylation 400 µM 2-deoxy-2-fluoro-L-fucose (2F; Carbosynth, Compton, Berkshire, UK) was added to the cell culture during production.(16) Antibodies were then purified on Protein A HiTrapHP columns (GE Healthcare Life Sciences, Little Chalfront,UK) using Akta-prime plus (GE Healthcare Life Sciences) and finally dialyzed against phosphate-buffered saline (PBS) overnight.

### Cell isolation and culture

Peripheral blood mononuclear cells (PBMCs) from healthy volunteers were isolated by Ficoll Paque density-gradient centrifugation. A mixed lymphocyte reaction was performed by culturing PBMC's from 2 donors in a 1:1 ratio in AIM V serum free medium. Proliferation was measured using a 3H thymidine incorporation assay. Regulatory macrophages were characterized by flow cytometry (Fortessa, BD) after staining for CD14-PE and CD206-APC (BD Biosciences)

### T cell transfer model of colitis

Female CB-17 SCID mice and wild type BALB/c mice were obtained from Harlan (Boxmeer, The Netherlands). Transfer of CD4+CD45RB^{hi} cells was performed as previously described.(18) In short, a single cell suspension was created by forcing spleens of wild type BALB/c mice through a cell strainer. Erythrocytes were removed by adding erythrocyte lysis-buffer followed by negative depletion of macrophages, B cells and CD8+ cells using magnetic beads. The remaining CD4+ cell enriched cell suspension was labelled and CD4+CD45RB^{hi} were isolated on a FACS sorter. Colitis was induced by injecting 2-3 x 10⁵ CD4+CD45RB^{hi} cells into SCID mice intraperitoneally. Weight was monitored twice weekly. After 3 to 4 weeks, mice were randomised into three groups and treatment was initiated. We conducted 2 independent experiments. For the first (suboptimal dose) experiment, animals received 25 µg per animal twice weekly. For the second (high dose) experiment, the mice were injected with 200 µg twice weekly. Additionally, the second experiment contained a control group of 5 mice that did not receive a T-cell transfer and therefore did not develop colitis (naive mice). All experimental procedures were approved by the local ethical committee.

### Flow Cytometry colons

Colons were cut in half longitudinally. One half was cut in pieces of 0,5 cm, washed 3 times with PBS, epithelium was separated with 5 mM EDTA, while shaking at 20 min at 37°C. Epithelial fractions were collected by pelleting. Colons were digested in HBSS FCS 2%, liberase 5 mg/ml DNAse 10 mg/ml for 40 min at 37°C with a magnetic stirrer. Cells were forced through a cell strainer and stained for CD64-PE, CD45-APC-Cy7, CD206-AF488, CD11b-PerCP (Biolegend) Ly6G-AF700 (BD Biosciences) Ly6C-APC (Bioscience) and DAPI and measured by flow cytometry (Fortessa BD).

### Cells and media

For the production of IgG we used the serum-free FreeStyle™ 293 Expression System (Invitrogen). Cells were cultured in 293 Freestyle® expression medium (Invitrogen, 12338018), at 37° C, 8% CO2, on a shaking platform with rotation speed 125 RMP and rotation radius of 25mm. For large volume cultures 0.125, 0.25, 0.5 or 1 L Erlenmeyer flasks (Corning, 431143, 431144, 431145 or 431147) were used, for small volume cultures non-treated 6-well culture plates were used (Corning, 3736). Cells were maintained as prescribed by Invitrogen. Cell count was measured using CASY cell counter (Roche Innovatis, Reutlingen, Germany). For the transfection of HEK freestyle cells Opti-MEM® (Gibco, Thermo Fisher Scientific, 31985062) and 293-Fectin™ (Invitrogen, Thermo Fisher Scientific, 12347019) was used.

### Production of high affinity antibodies

### Transfection of IgG

Further transfection procedure was according to manufacturer's protocol (Invitrogen) and modified for IgG production as described by Vink et al 39. Cell supernatant was harvested after 5 days, cells were pelleted by centrifugation at maximum speed (≥ 4000 g) and supernatant was filtered using a 0.45nm puradisc syringe filter (Whatmann, GE Healthcare, 10462100).

### Glyco-engineering

Where indicated, galactose substrate, D-galactose (Sigma, G0750-5G), was added to the medium before transfection. The decoy substrates for fucosylation, 2-deoxy-2-fluoro-1-fucose (2FF) (Carbosynth, MD06089) and galactosylation 2-deoxy-2-fluoro-d-galactose (2FG) (Carbosynth, MD04718) were added 4 hours post transfection. All carbohydrates were diluted in ddH2O and filtered using a 0.2nm puradisc syringe filter (Whatmann, GE Healthcare, 10462200). Percentages of co-transfection of enzyme (glycosyltransferase or RMD) vectors ware always relative to total DNA. While titrating the enzyme vector, the amount of vector encoding for the antibody was kept constant, and an empty expression vector was added to keep the amount of total DNA constant. Ex vivo sialylation was performed with recombinant human α-2,6-sialyltransferase (Roche, 07012250103) and cytidine-5'-monophospho-N-acetylneuraminic acid (CMP-NANA) (Roche, 05974003103). Antibody, enzyme and substrate were mixed in a 20:1:10 w/w ratio in PBS at pH 7.4, incubated for 12 hours at 37° C, subsequently additional CMP-NANA was added to the mixture to a final ratio of 20:1:20 for antibody, enzyme and substrate respectively and further incubated for 12 hours at 37°C. After ex vivo sialylation to remove the excess enzyme and CMP-NANA, the antibodies were re-purified on a protein A (WT IgG1) HiTrap HP column (GE Life Sciences) as described previously.

### Reducing core-fucosylation

We first tested the potential of the bacterial enzyme GDP-6-deoxy-D-lyxo-4-hexulose reductase (RMD)32 to block the de novo production of fucose in HEK cells. Co-transfection of RMD together with IgG1 indeed resulted in decreased incorporation of core fucose in the N297-Fc glycan, from 94% to 30% with an optimum at 5% RMD vector. An alternative method to decrease fucosylation in IgG is to use the decoy substrate 2-deoxy-2-fluoro-1-fucose (2FF). 14,33 Addition of 2FF to the culture media resulted in reduced incorporation of fucose in the IgG-Fc glycan down to 19% (Fig. 3B) at an optimum of 0.4 mM 2FF. Combining both methods resulted in no further reduction beyond that seen with 2FF alone. 2FF treatment does not affect the level of other glycan traits, galactosylation, sialylation and bisection, and IgG yield, and thus 0.4mM 2FF was used for lowering core-fucosylation of IgG1, resulting in IgG1 with the most abundant glycan forms consisting of afucosylated species.

### Increased GlcNAc (bisection)

The human GlcNAc transferase III, beta-1,4-mannosyl-glycoprotein 4-beta-N-acetylglucosaminyltransferase (GNTIII) enzyme is known to be responsible for the addition of GlcNAc to complex bi-antennary glycans. As low as 1% addition of DNA encoding for GNTIII to the transfection mixture resulted in maximally 57% incorporation of bisecting GlcNAc in the IgG-Fc. The co-transfection of GNTIII resulted in a slight increase in galactosylation and decrease in fucosylation, but no significant change in antibody yield. A slight increase in hybrid and high mannose glycoforms (<5% of total glycoforms) was observed upon this treatment. These glycoforms are not present on IgG in serum but are known to occur within monoclonal antibody production. The most abundant glycoforms of IgG1 produced with 1% GNTIII consisted of bisected IgG1-glycoforms.

### Decreasing galactosylation

To reduce the level of galactosylation of HEK-produced IgG, we identified the galactose analog 2-deoxy-2-fluoro-d-galactose (2FG) as a specific blocker of galactosylation. By addition of 1 mM 2FG to the culture medium, incorporation of galactose into the IgG1 N297-Fc glycan was reduced down to 8%. While a slight effect on fucosylation was observed, this method still resulted in almost exclusively fucosylated IgG1 without galactose. Addition of an even higher concentration decreased IgG-yields without further reduction of the degree of galactosylation.

### Increasing galactosylation

In vivo, galactose can be added to N-linked glycans by several different galactosyl transferases. In B-cells β-1,4-galactosyltransferase 1 (B4GALT1) is expressed,17 with β-1,4-galactosyltransferase 2 (B4GALT2) having very similar if not identical substrate preference. When comparing both B4GALT1 and B4GALT2 for inducing galactosylation we found that inclusion of 1% DNA encoding for B4GALT1 to the transfection mix induced galactosylation up to 70% and performed better than B4GALT2 with 42%. We noted that higher dosages reduced the yield of IgG production.
In addition to increasing the level of IgG1-Fc galactosylation through enzyme manipulation, we also tested the effect of substrate availability on galactosylation. By adding D-Galactose to the medium a gradual increase in galactosylation was observed, that was further increased by co-transfection of galactosyl transferases. Except for a slight expected increase in sialylation we did not see an effect on the other glycan traits when increasing galactosylation. An optimum was reached by co-transfection of 1% B4GALT1 and 5 mM D-galactose addition, resulting in 85% galactosylation.

### Increasing sialylation

As sialic acid is the terminal sugar group on the complex glycan, to increase the level of sialylation a high level of galactosylation is needed. The optimal tools for the addition of galactose (B4GALT1 co-transfection and D-galactose addition, were combined with co-transfection of β-galactoside alpha-2,6-sialyltransferase 1 (ST6GALT). This enzyme ensures the correct α-2,6-linkage found in human IgG. Sialylation levels gradually increased by increasing the amount of ST6GALT in the co-transfection mix. Higher levels of ST6GALT vector affected IgG production levels negatively and also slightly affected galactosylation levels while leaving fucosylation and bisection unchanged. To further increase sialylation of the IgG, the highly galactosylated and sialylated IgG was treated ex-vivo with recombinantly produced ST6GALT and cytidine-5'-monophospho-N-acetylneuraminic acid (CMP-NANA) substrate, which resulted in 74% sialylation. By transfection only, optimal ST6GALT concentration was determined to be 2.5% vector relative to antibody vector, reaching 33% sialylation, or roughly half of the available galactose residues, with both mono- and disialylated glycan species present in similar amounts. By ex-vivo sialylation, the level of disialylated species was further increased, with roughly 90% of available galactose residues covered.

### Biosensor analysis

All biotinylated FcγR were spotted using a Continuous Flow Microspotter (Wasatch Microfluidics, Salt Lage City, UT) onto a single SensEye G-streptavidin sensor (Ssens, Enschede, Netherlands) allowing for binding affinity measurements of each antibody to all FcγR simultaneously on the IBIS MX96 (IBIS Technologies, Enschede, Netherlands) as described (Schasfoort et al. 2013, Anal Biochem. 2013;439(1):4-6). The biotinylated FcγRs were spotted in three-fold dilutions, ranging from 30nM to 1nM in PBS 0.0075% Tween-80 (Amresco, Solon, OH, USA), pH 7.4. The IgGs were then injected over the IBIS at 1.5 dilution series starting at 5.9 nM until 506.25 nM in PBS in 0.075% Tween-80. Regeneration with acid buffer (10mM Gly-HCl, 0.075% Tween 80, pH2.5) was carried out after every sample. Calculation of the dissociation constant (kD) was done using an equilibrium analysis by linear intrapolation to Rmax=500.Analysis and calculation of all binding data was carried out with Scrubber software version 2 (Biologic Software, Campbell, Australia). Measurements were carried out at room temperature.

### Results

### Afucosylation of adalimumab enhances regulatory macrophage induction and immunosuppressive properties of the antibody in vitro.

We have previously shown that the therapeutic efficacy of anti-TNFs in patients with Crohn's disease is reflected by their activity in vitro in a mixed lymphocyte reaction (MLR).(1) We treated MLRs with a serial dilution of adalimumab or afucosylated adalimumab. To mimic physiological circumstances where adalimumab competes for receptor binding with endogenous IgG, human IgG was added to every condition to reach a total antibody concentration of 1 µg/ml per well. Our data show that afucosylated adalimumab suppressed T-cell proliferation in a dose-dependent manner and was more potent than fucosylated adalimumab. (Fig.1A) In addition, we measured the percentage of CD206+ regulatory macrophages within the MLR by flow cytometry. Indeed, afucosylated adalimumab induced more CD206+ macrophages than fucosylated adalimumab, again this effect was dose-dependent. (Fig. 1B)

### Afucosylated chimeric human IgG1 anti-mouse TNF is more effective in the T-cell transfer model of colitis.

To study the immunosuppressive capacity of afucosylated anti-TNF in vivo, we used the T-cell transfer colitis mouse model. SCID mice lacking functional T and B cells, are reconstituted with effector T cells. Due to a lack of regulatory T cells, the animals will develop a chronic colitis that recapitulates some of the hallmarks of inflammatory bowel disease in humans. Two independent experiments were performed to study different dosing regimens. In the first experiment, the animals were treated with a suboptimal dose of 25 ug anti-TNF per mouse twice weekly. In the second experiment, we treated the mice with a high dose of 200 ug anti-TNF per mouse twice weekly. Loss of weight is one of the clinical signs that illustrates disease activity. In both experiments, weight loss was less severe in the mice receiving afucosylated anti-TNF compared to fucosylated anti-TNF. (Fig. 2A and Fig. 2C) The most important outcome parameter in this model is histopathological scoring of the colon. In the experiment were mice received high dose anti-TNF, both drugs reduced histopathological colitis scores to such an extent that improvement is no longer possible. (Fig. 2D) This indicates that both drugs have the ability to improve colitis. However, in the suboptimal dosing experiment, only the afucosylated anti-TNF antibody significantly improved the severity of the colitis. (Fig. 2B)
We have shown previously that anti-TNF induces CD206+ regulatory macrophages in vitro and in vivo in humans.(5, 6) To study this in mice, we used half of the colons to characterize macrophage populations by flow cytometry. To select for macrophages, we gated on live CD45+CD11b+Ly6G-CD64+ myeloid cells. Within this macrophage gate, we subsequently gated for CD206^{hi}Ly6C^{lo} macrophages and CD206^{lo}Ly6C^{hi} to select for regulatory (M2) and pro-inflammatory (M1) macrophages respectively. In line with our human in vitro data shown above, we found that afucosylated anti-TNF induced more CD206^{high}Ly6C^{low} macrophages (Fig. 3A) and significantly increased the M2/M1 ratio compared to normal anti-TNF (Fig. 3B).
Altogether, these data provide evidence that afucosylation of anti-TNF results in a superior antibody for the treatment of IBD or colitis in vivo. Based on extensive glycoenginering efforts, where we see even better binding of defucosylated and highly galactosylated glycovariants (Figure 4), we expect that that these glycoforms will outperform defucosylated anti-TNF antibodies.

### REFERENCES

1. Derkx B, et al. (1993) Tumour-necrosis-factor antibody treatment in Crohn's disease. Lancet 342(8864): 173-174.
2. Rutgeerts P, et al. (2006) Scheduled maintenance treatment with infliximab is superior to episodic treatment for the healing of mucosal ulceration associated with Crohn's disease. Gastrointestinal endoscopy 63(3):433-442; quiz 464.
3. Rutgeerts P, et al. (2012) Adalimumab induces and maintains mucosal healing in patients with Crohn's disease: data from the EXTEND trial. Gastroenterology 142(5):1102-1111 e1102.
4. Hebuterne X, et al. (2013) Endoscopic improvement of mucosal lesions in patients with moderate to severe ileocolonic Crohn's disease following treatment with certolizumab pegol. Gut 62(2):201-208.
5. Vos AC, et al. (2011) Anti-tumor necrosis factor-alpha antibodies induce regulatory macrophages in an Fc region-dependent manner. Gastroenterology 140(1):221-230.
6. Vos AC, et al. (2012) Regulatory macrophages induced by infliximab are involved in healing in vivo and in vitro. Inflammatory bowel diseases 18(3):401-408.
7. Mosser DM & Edwards JP (2008) Exploring the full spectrum of macrophage activation. Nature reviews. Immunology 8(12):958-969.
8. Sutterwala FS, Noel GJ, Clynes R, & Mosser DM (1997) Selective suppression of interleukin-12 induction after macrophage receptor ligation. The Journal of experimental medicine 185(11):1977-1985.
9. Sutterwala FS, Noel GJ, Salgame P, & Mosser DM (1998) Reversal of proinflammatory responses by ligating the macrophage Fcgamma receptor type I. The Journal of experimental medicine 188(1):217-222.
10. McRae BL, et al. (2016) Fc Receptor-mediated Effector Function Contributes to the Therapeutic Response of Anti-TNF Monoclonal Antibodies in a Mouse Model of Inflammatory Bowel Disease. J Crohns Colitis 10(1):69-76.
11. Krapp S, Mimura Y, Jefferis R, Huber R, & Sondermann P (2003) Structural analysis of human IgG-Fc glycoforms reveals a correlation between glycosylation and structural integrity. J Mol Biol 325(5):979-989.
12. Shields RL, et al. (2002) Lack of fucose on human IgG1 N-linked oligosaccharide improves binding to human Fcgamma RIII and antibody-dependent cellular toxicity. The Journal of biological chemistry 277(30):26733-26740.
13. Goede V, et al. (2014) Obinutuzumab plus chlorambucil in patients with CLL and coexisting conditions. The New England journal of medicine 370(12):1101-1110.
14. van Oers MH (2012) CD20 antibodies: type II to tango? Blood 119(22):5061-5063.
15. Kruijsen D, et al. (2013) Intranasal administration of antibody-bound respiratory syncytial virus particles efficiently primes virus-specific immune responses in mice. Journal of virology 87(13):7550-7557.
16. Kapur R, et al. (2014) A prominent lack of IgG1-Fc fucosylation of platelet alloantibodies in pregnancy. Blood 123(4):471-480.
17. Vink T, Oudshoorn-Dickmann M, Roza M, Reitsma JJ, & de Jong RN (2014) A simple, robust and highly efficient transient expression system for producing antibodies. Methods 65(1):5-10.
18. Read S & Powrie F (2001) Induction of inflammatory bowel disease in immunodeficient mice by depletion of regulatory T cells. Current protocols in immunology / edited by John E. Coligan ... [et al.] Chapter 15:Unit 15 13.

## Claims

1. A modified anti-TNF antibody having an affinity for the 158V polymorfism of FcγRIIIa of more than 7.7 * 10⁶ KA (M⁻¹) as determined using biosensor analysis.

2. The modified anti-TNF antibody according to claim 1, being an afucosylated anti-TNF antibody comprising a glycan moiety at Asn297 with an amount of fucose of 60% or less of the total amount of oligosaccharides.

3. The modified anti-TNF antibody according to claim 1 or the afucosylated anti-TNF antibody of claim 2, wherein said glycan moiety is high-galactosylated.

4. The modified anti-TNF antibody according to claim 1 or the afucosylated anti-TNF antibody of claim 2 or 3, which induces a greater number of regulatory macrophages than a control fucosylated anti-TNF antibody.

5. The modified anti-TNF antibody according to claim 1 or the afucosylated anti-TNF antibody of any one of claims 2-4, which comprises complementarity determining regions (CDRs) of the antibody adalimumab, infliximab, certolizumab, or golimumab.

6. The modified anti-TNF antibody according to claim 1 or the afucosylated anti-TNF antibody of any one of claims 2-5, wherein said anti-TNF antibody is selected from the group consisting of adalimumab, etanercept, golimumab, and infliximab and a biosimilar form of any of adalimumab, etanercept, golimumab, and infliximab.

7. A pharmaceutical composition comprising the modified anti-TNF antibody according to claim 1 or the afucosylated anti-TNF antibody according to any one of claims 2-6 and a pharmaceutically acceptable carrier.

8. The pharmaceutical composition according to claim 7, wherein the amount of modified anti-TNF antibody or afucosylated anti-TNF antibody is at least 25% of the total number of anti-TNF antibodies.

9. The modified anti-TNF antibody according to claim 1 or the afucosylated anti-TNF antibody of any one of claims 2-6, or the pharmaceutical composition according to claim 7 or 8 for use in the treatment of a disease.

10. The modified anti-TNF antibody, afucosylated anti-TNF antibody or the pharmaceutical composition according to claim 9, wherein said disease is inflammatory bowel disease (IBD).

11. The modified anti-TNF antibody, afucosylated anti-TNF antibody or the pharmaceutical composition according to any one of claims 8-10, wherein said treatment comprises the administration of said afucosylated anti-TNF antibody between once or twice weekly and once every 10 weeks.

12. The modified anti-TNF antibody, afucosylated anti-TNF antibody or the pharmaceutical composition according to any one of claims 8-11, wherein said treatment comprises the administration of said afucosylated anti-TNF antibody at a dosage between 0.1-20 mg/kg, more preferably between 1.25 and 10 mg/kg.

13. A host cell expressing the modified anti-TNF antibody as defined in claim 1 or the afucosylated anti-TNF antibody as defined in any one of claims 2-6.

14. A method for preparing an afucosylated anti-TNF antibody as defined in any one of claims 2-6, the method comprising the steps of
- providing a host cell with a nucleic acid molecule encoding an anti-TNF antibody amino acid sequence;
- expressing the anti-TNF antibody in the host cell; and
- harvesting, purifying and/or isolating said afucosylated antibody,
wherein said host cell has a reduced capacity to fucosylate and/or wherein said host cell expresses said anti-TNF antibody in the presence of an effective amount of a fucosylation inhibitor.


SEQUENCE LISTING

<110> Academisch Medisch Centrum

<120> NON-FUCOSYLATED ANTI-TNF ANTIBODY AND USE THEREOF IN THE TREATMENT OF
IBD

<130> INV-00559

<160> 1

<170> BiSSAP 1.3.6

<210> 1
<211> 25
<212> DNA
<213> Artificial Sequence

<220>
<223> 158V

<400> 1
ctgaagacac atttttactc ccaac                                          25